# EUROPÄISCHE PATENTSCHRIFT

(11) **EP 2 265 243 B1**
(45) Veröffentlichungstag und Bekanntmachung des Hinweises auf die Patenterteilung: **21.06.2017**
(21) Anmeldenummer: 09723608.7
(22) Anmeldetag: 04.02.2009
(51) Int. Cl.: A61K 8/34, A61K 8/42, A61K 8/49, A61Q 15/00, A61Q 19/00

(54) **KÜHLENDE KOSMETISCHE ODER DERMATOLOGISCHE ZUBEREITUNGEN MIT EINEM GEHALT AN (1R,2S,5R)-2-ISOPROPYL-5-METHYL-N-(2-(PYRIDYN-2-YL)ETHYL-CYCLOHEXANCARBOXAMID UND/ODER (1R,2S,5R)-N-(4-(CYANOMETHYL)PHENYL)-2-ISOPROPYL-5- METHYLCYCLOHEXANCARBOXAMID MIT MENTHOXYPROPANDIOL**
COOLING COSMETIC OR DERMATOLOGICAL PREPARATIONS HAVING A CONTENT OF (1R,2S,5R)-2-ISOPROPYL-5-METHYL-N-(2-(PYRIDYNE-2-YL)ETHYL-CYCLOHEXANE CARBOXAMIDE AND/OR (1R,2S,5R)-N-(4-(CYANOMETHYL)PHENYL)-2-ISOPROPYL-5- METHYLCYCLOHEXANE CARBOXAMIDE COMPRISING MENTHOXYPROPANE DIOL
PRÉPARATIONS COSMÉTIQUES OU DERMATOLOGIQUES RAFRAÎCHAISSANTES AYANT UNE TENEUR EN (1R,2S,5R)-2-ISOPROPYL-5-MÉTHYL-N-(2-(PYRIDYN-2-YL)ÉTHYL-CYCLOHEXANCARBOXAMIDE ET/OU EN (1R,2S,5R)-N-(4-(CYANOMÉTHYL)PHÉNYL)-2-ISOPROPYL-5- MÉTHYLCYCLOHEXANCARBOXAMIDE ET EN MENTHOXYPROPANDIOL

(30) Priorität: 20.03.2008 DE 102008015424
(43) Veröffentlichungstag der Anmeldung: 29.12.2010
(73) Patentinhaber: Beiersdorf AG, 20253 Hamburg (DE)
(72) Erfinder: KOLBE, Ludger, 21255 Dohren (DE); ECKERT, Julia, 20249 Hamburg (DE); NEUFANG, Gitta, 20149 Hamburg (DE); WÖHRMANN, Michael, 22525 Hamburg (DE); KNAUPMEIER, Stefanie, 32108 Bad Salzuflen (DE); HÖLTKEMEIER, Stefanie, 22391 Hamburg (DE)
(74) Vertreter: Hartmann, Jost
(86) Internationale Anmeldenummer: PCT/EP2009/000718
(87) Internationale Veröffentlichungsnummer: WO 2009/115164

(56) Entgegenhaltungen:
- EP-A- 1 430 880
- WO-A-00/62737
- WO-A-2005/049553
- WO-A-2007/019719
- WO-A2-2006/103401
- US-A- 4 459 425
- US-A1- 2003 235 545

## Beschreibung

Die vorliegende Erfindung betrifft kühlende kosmetische und dermatologische Zubereitungen, insbesondere hautpflegende kosmetische und dermatologische Zubereitungen.

Die Kühlung der Haut kann etwas sehr Angenehmes sein. Dies ist besonders so, wenn die Haut sich heiß anfühlt, schmerzt, juckt oder frisch rasiert ist. Oft ist dies verbunden mit Sonnenbrand oder -bad. Bei Insektenstichen oder entzündlicher Haut, Akne kann Kühlung der Haut ebenfalls lindernd empfunden werden.

Bisher gibt es verschiedene Coolingsubstanzen, die auf der Haut ein angenehmes Frischegefühl hervorrufen. So wird beispielsweise Menthol eingesetzt, um in After Shave Formulierungen ein Frischegefühl zu bewirken. Menthol besitzt jedoch schleimhautreizende Eigenschaften und kann weitere Hautirritationen verursachen, was zu einem Brennen der Haut führt. Darüber hinaus besitzt Menthol einen starken Eigengeruch und kann zu Unverträglichkeiten im Augenbereich führen.

Es hat nicht an Versuchen gefehlt, die Kühlung der Haut durch Auftragen einer Zubereitung zu bewirken. Es wurde auch versucht, Zubereitungen zu finden, die kühlen.

Dies wurde besonders für Haut nach der Rasur oder die Haut nach dem Sonnenbrand- oder -bad versucht. Bekannte Produkte werden als After-Shave oder After-Sun bezeichnet.

Es war eine Aufgabe der vorliegenden Erfindung, den Übelständen des Standes der Technik abzuhelfen und Kühlung der Haut ermöglichende Zubereitungen zur Verfügung zu stellen, welche einfach herzustellen sind, keine Reizwirkung auf Haut oder Schleimhäute ausüben, geruchsneutral sind, sowie bei bestimmungsgemäßer Anwendung angenehme Kühlung spenden.

Es war jedoch erstaunlich und für den Fachmann nicht vorhersehbar, daß kühlende topische kosmetische oder dermatologische Zubereitungen
mit einem Gehalt an
Stoff A): (1R,2S,5R)-2-Isopropyl-5-methyl-N-(2-(pyridin-2-yl)ethyl-cyclohexancarboxamid und/oder
   (1R,2S,5R)-N-(4-(Cyanomethyl)-phenyl)-2-isopropyl-5-methylcyclohexancarboxamid und
Stoff B): Menthoxypropandiol.
dadurch gekennzeichnet,
daß sie in Form von O/W- Emulsionen vorliegen
daß sie 0,001-10 Gew.-%, besonders bevorzugt 0,01-1 Gew.-%, an Stoff A) enthalten, bezogen auf die Gesamtzusammensetzung der Zubereitungen und
daß das Verhältnis Stoff A) zu Stoff B) 1 bis 20, besonders bevorzugt 1,5 bis 15, die Nachteile des Standes der Technik beseitigen.

Diese juckreizreduzierenden Coolingsubstanzen können auch in Kombination mit anderen Coolingsubstanzen eingesetzt werden.

Beispiele für andere Coolingsubstanzen sind Menthol, Menthone, Ethyl Menthane Carboxamido acetate (WS-5), Isopulegol (Coolact P), Menthanediol (Coolact 38D), N,2,3-trimethyl-2-isopropylbutanamide (WS-23), Ethyl Menthane Carboxamide (WS-3), menthone glycerine acetal (Frescolat MGA), mono-menthyl succinate (Physcool) oder 2-Isopropyl-5-methyl-cyclohexane-carbonyl-amido-propionsäure-methylester.

Die Zubereitungen sind einfach zu formulieren und stellen keine großen Anforderungen an Herstellungsvorgänge.

Die Cooling Substanzen werden in Polyolen vorgelöst und bei ca. 40°C zur Emulsion vor dem Abkühlen auf Raumtemperatur zugegeben.

Die Erfindung umfasst auch die Verwendung von kosmetischen oder dermatologischen Zubereitungen
mit einem Gehalt an
Stoff A): (1R,2S,5R)-2-Isopropyl-5-methyl-N-(2-(pyridin-2-yl)ethyl-cyclohexancarboxamid und/oder
   (1 R,2S,5R)-N-(4-(Cyanomethyl)-phenyl)-2-isopropyl-5-methylcyclohexancarboxamid und
Stoff B): Menthoxypropandiol.
dadurch gekennzeichnet,
daß sie in Form von O/W-Emulsionen vorliegen
daß sie 0,001-10 Gew.-%, besonders bevorzugt 0,01-1 Gew.-%, an Stoff A) enthalten, bezogen auf die Gesamtzusammensetzung der Zubereitungen und
daß das Verhältnis Stoff A) zu Stoff B) 1 bis 20, besonders bevorzugt 1,5 bis 15 ist,
zur Erzeugung eines Kältegefühls auf der Haut.

Weiter besonders bevorzugt ist es, wenn die Zubereitungen Antioxidantien enthalten. Weiter besonders bevorzugt ist es, wenn die Zubereitungen Vitamine enthalten. Weiter besonders bevorzugt ist es, wenn die Zubereitungen Alkohole enthalten. Weiter besonders bevorzugt ist es, wenn die Zubereitungen Antitranspirantien enthalten. Weiter besonders bevorzugt ist es, wenn die Zubereitungen Deodorantien enthalten.

Zur Anwendung werden die kosmetischen und dermatologischen Zubereitungen erfindungsgemäß in der für Kosmetika üblichen Weise auf die Haut in ausreichender Menge aufgebracht.

Die kosmetischen und dermatologischen Zubereitungen können erfindungsgemäß kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an üblichen Antioxidantien ist im Allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der vorgenannten Antioxidantien (eine oder mehrere Verbindungen) in den Zubereitungen beträgt vorzugsweise 0,001 bis 30 Gew.-%, besonders bevorzugt 0,05-20 Gew.-%, insbesondere 1-10 Gew.-%, bezogen auf das Gesamtgewicht der Zubereitung.

Es können als Lösungsmittel verwendet werden:
- Wasser oder wässrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Polyethylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und dermatologische Anwendungen geeigneten oder gebräuchlichen Antioxidantien verwendet werden.

Ganz besonders vorteilhaft im Sinne der vorliegenden Erfindung können wasserlösliche Antioxidantien eingesetzt werden, wie beispielsweise Vitamine, z. B. Ascorbinsäure und deren Derivate - insbesondere Ascorbylpalmitat, Na- und Mg-Ascorbylphosphat und Ascorbylacetat - sowie Rutinsäure und deren Derivate insbesondere alpha Glucosylrutin, Quercetin und Isoquercetin.

Besonders bevorzugte Antioxidantien sind ferner Vitamin E und dessen Derivate (insbesondere Vitamin E-Acetat), Vitamin A und dessen Derivate (insbesondere Vitamin-A-Palmitat) sowie Carnosin, Butylhydroxytoluol, Butylhydroxyanisol, beta-Alanin sowie Carotinoide (insbesondere beta-Carotin) und Phytoen.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wässrigen Phase einzusetzen.

Vorteilhaft können die Lichtschutzformulierungen erfindungsgemäß weitere Substanzen enthalten, die UV-Strahlung im UVB-Bereich absorbieren, wobei die Gesamtmenge der Filtersubstanzen z. B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| Tagescreme Gew.-% | |
|---|---|
| Glycerylstearatcitrat | 3 |
| Cetylalkohol | 2 |
| Glycerylstearat | 1 |
| Cyclomethicon 3Butylmethoxydibenzoylmethan (Parsol(R) 1789) | 2 |
| Ethylhexyl-2-cyano-3,3-diphenylacrylat (Octocrylene) | 5 |
| Ethylhexylmethoxycinnamat | 2 |
| Menthoxypropandiol | 1 |
| (1R,2S,5R)-2-Isopropyl-5-methyl-N-(2-(pyridyn-2-yl)ethyl-cyclohexancarboxamid | 0,1 |
| C12-15 Alkylbenzoat | 3 |
| Carbomer | 0,2 |
| Natriumascorbylphosphate | 0,2 |
| 3-(4-Hydroxy-3-methoxyphenyl)-1-(4-hydroxyphenyl)-propan-1-on | 0,5 |
| Glycerin | 8 |
| (1R,2S,5R)-N-(4-(Cyanomethyl)-phenyl)-2-isopropyl-5-methylcyclohexancarboxamid | 0,02 |
| Parfüm | 0,3 |
| Natronlauge | q. s. |
| Wasser | ad 100 |
| pH 5 | |

| Pflegecreme | Gew.-% |
|---|---|
| Glycerylstearat, selbstemulgierend | 5 |
| Stearylalkohol | 3 |
| Stearinsäure | 1 |
| (1R,2S,5R)-N-(4-(Cyanomethyl)-phenyl)-2-isopropyl-5-methylcyclohexancarboxamid | 0.08 |
| Dimeticonol | 0,5 |
| Cyclomethicone | 4 |
| Octyldodecanol | 3 |
| Hydrierte Kokosfettglyceride | 2 |
| Menthoxypropandiol | 0,4 |
| 3-(4-Hydroxy-3-methoxyphenyl)-1-(4-hydroxyphenyl)-propan-1-on | 0,2 |
| Natrium Stärke octenylsuccinat | 1 |
| Carbomer | 0,2 |
| (1R,2S,5R)-2-Isopropyl-5-methyl-N-(2-(pyridyn-2-yl)ethyl-cyclohexancarboxamid | 0,06 |
| Glycerin | 9 |
| EDTA | 0,1 |
| Parfum | 0,3 |
| Tocopherylacetat | 0,5 |
| Coenzym Q 10 | 0,1 |
| Methylpropandiol | 1 |
| Natronlauge | q. s. |
| Wasser | ad 100 |
| pH: 7 | |

| Kühlende Hautcreme | Gew.-% |
|---|---|
| Polyglyceryl-3 Methylglucosedistearat: | 2 |
| Sorbitanstearat | 2 |
| Stearylalkohol | 2 |
| Dimethicone | 2 |
| C12-15 Alkylbenzoat | 2 |
| Caprylsäure/Caprinsäuretriglyceride | 3 |
| Cyclomethicon | 4 |
| Ethylhexyl triazon | 1 |
| 2,2'-Methylen-bis-(6-(2H-benzotriazol-2-yl)-4-(1,1,3,3-tetramethylbutyl)-phenol) | 2 |
| Ethylhexyl-2-cyano-3,3-diphenylacrylat (Octocrylene) | 3 |
| 3-(4-Hydroxy-3-methoxyphenyl)-1-(4-hydroxyphenyl)-propan-1-on | 0,5 |
| Carrageenan | 0,1 |
| Vernetztes Alkylacrylat (Alkylacrylate Crosspolymer) | 0,1 |
| (1 R,2S,5R)-N-(4-(Cyanomethyl)-phenyl)-2-isopropyl-5-methylcyclohexancarboxamid | 0,05 |
| Menthoxypropandiol | 0,8 |
| Glycerin | 8 |
| Ethylhexylglycerin | 0,4 |
| (1R,2S,5R)-2-Isopropyl-5-methyl-N-(2-(pyridyn-2-yl)ethyl-cyclohexancarboxamid | 0,06 |
| Ethylparaben | 0,2 |
| Phenoxyethanol | 0,4 |
| Additive (Nylon, Stärkephosphat) | 2 |
| Citronensäure, Natriumsalz | q. s. |
| Wasser | ad 100 |
| pH: 6 | |

| Sonnenschutzcreme mit Cooling Effekt | Gew.-% |
|---|---|
| Glycerylstearat | 3 |
| PEG-40-Stearat | 1 |
| Cetylalkohol | 2 |
| C12-15 Alkylbenzoat | 2 |
| Dicaprylylether | 2 |
| Octyldodecanol | 2 |
| Cyclomethicon | 2 |
| Myristylmyristat | 1 |
| (1R,2S,5R)-N-(4-(Cyanomethyl)-phenyl)-2-isopropyl-5-methylcyclohexancarboxamid | 0,04 |
| Ethylhexylmethoxycinnamat | 6 |
| Phenylbenzimidazolsulfonsäure | 1 |
| Aniso Triazin (2,4-Bis-1{[4-(2-Ethyl-hexyloxy)-2-hydroxy]-phenyl}-6-(4-methoxyphenyl)-1,3,5-triazin) | 2 |
| 3-(4-Hydroxy-3-methoxyphenyl)-1-(4-hydroxyphenyl)-propan-1-on | 0,2 |
| Carragenan | 0,2 |
| Carbomer | 0,2 |
| Glycerin | 7 |
| Butylenglycol | 3 |
| (1R,2S,5R)-2-Isopropyl-5-methyl-N-(2-(pyridyn-2-yl)ethyl-cyclohexancarboxamid | 0,6 |
| Menthoxypropandiol | 1,5 |
| Parfum | q. s. |
| Natronlauge | q. s. |
| Wasser ad 100 | |
| pH: 7 | |

| After Shave Balsam | Gew.-% |
|---|---|
| Triceteareth-4 phosphat | 1 |
| Clyclomethicone | 3 |
| C12-15 Alkylbenzoat | 2 |
| Menthoxypropandiol | 1 |
| Distärke phosphat | 1 |
| (1R,2S,5R)-2-Isopropyl-5-methyl-N-(2-(pyridyn-2-yl)ethyl-cyclohexancarboxamid | 0,1 |
| Xanthan Gum | 0,1 |
| Methylparaben | 0,2 |
| Phenoxyethanol | 0,4 |
| (1R,2S,5R)-N-(4-(Cyanomethyl)-phenyl)-2-isopropyl-5-methylcyclohexancarboxamid | 0,04 |
| Glycerin | 6 |
| EDTA | 0,1 |
| Parfum | 0,3 |
| Natronlauge | q. s. |
| Wasser | ad 100 |
| pH: 7 | |

## Patentansprüche

1. Kühlende topische kosmetische oder dermatologische Zubereitungen
mit einem Gehalt an
Stoff A): (1R,2S,5R)-2-Isopropyl-5-methyl-N-(2-(pyridin-2-yl)ethyl-cyclohexancarboxamid und/oder
(1R,2S,5R)-N-(4-(Cyanomethyl)-phenyl)-2-isopropyl-5-methylcyclohexancarboxamid und
Stoff B): Menthoxypropandiol.
**dadurch gekennzeichnet,**
**daß** sie in Form von O/W- Emulsionen vorliegen
**daß** sie 0,001-10 Gew.-%, besonders bevorzugt 0,01-1 Gew.-%, an Stoff A) enthalten, bezogen auf die Gesamtzusammensetzung der Zubereitungen und
**daß** das Verhältnis Stoff A) zu Stoff B) 1 bis 20, besonders bevorzugt 1,5 bis 15 ist.

2. Verwendung von kosmetischen oder dermatologischen Zubereitungen
mit einem Gehalt an
Stoff A): (1R,2S,5R)-2-Isopropyl-5-methyl-N-(2-(pyridin-2-yl)ethyl-cyclohexancarboxamid und/oder
(1R,2S,5R)-N-(4-(Cyanomethyl)-phenyl)-2-isopropyl-5-methylcyclohexancarboxamid und
Stoff B): Menthoxypropandiol.
**dadurch gekennzeichnet,**
**daß** sie in Form von O/W-Emulsionen vorliegen
**daß** sie 0,001-10 Gew.-%, besonders bevorzugt 0,01-1 Gew.-%, an Stoff A) enthalten, bezogen auf die Gesamtzusammensetzung der Zubereitungen und
**daß** das Verhältnis Stoff A) zu Stoff B) 1 bis 20, besonders bevorzugt 1,5 bis 15 ist,
zur Erzeugung eines Kältegefühls auf der Haut.

## Claims

1. Cooling topical cosmetic or dermatological preparations
having a content of
substance A): (1 R,2S,5R)-2-isopropyl-5-methyl-N-(2-(pyridine-2-yl)ethylcyclohexane carboxamide
and/or
(1 R,2S,5R)-N-(4-cyanomethyl)phenyl)-2-isopropyl-5-methylcyclohexane carboxamide and
substance B): menthoxypropanediol
**characterized in that**
they are present in the form of O/W emulsions
that they comprise 0.001-10% by weight, particularly preferably 0.01-1% by weight of substance A, based on the total composition of the preparations and
that the ratio of substance A) to substance B) is 1:20, particularly preferably 1.5:15.

2. Use of cosmetic or dermatological preparations
having a content of
substance A): (1 R,2S,5R)-2-isopropyl-5-methyl-N-(2-(pyridine-2-yl)ethylcyclohexane carboxamide
and/or
(1 R,2S,5R)-N-(4-cyanomethyl)phenyl)-2-isopropyl-5-methylcyclohexane carboxamide and
substance B): menthoxypropanediol
**characterized in that**
they are present in the form of O/W emulsions
that they comprise 0.001-10% by weight, particularly preferably 0.01-1% by weight of substance A, based on the total composition of the preparations and
that the ratio of substance A) to substance B) is 1:20, particularly preferably 1.5:15 for generating a cooling feel on the skin.

## Revendications

1. Préparations cosmétiques ou dermatologiques, topiques, rafraîchissantes, présentant une teneur en
substance A) :
(1 R,2S,5R)-2-isopropyl-5-méthyl-N-(2-(pyridin-2-yl)éthylcyclohexanecarboxamide et/ou
(1 R,2S,5R)-N-(4-(cyanométhyl)-phényl)-2-isopropyl-5-méthylcyclohexanecarboxamide et
substance B) : menthoxypropanediol
**caractérisées en ce qu'**elles se trouvent sous forme d'émulsions H/E, **en ce qu'**elles contiennent 0,001-10% en poids, de manière particulièrement préférée 0,01-1% en poids, de substance A), par rapport à la composition totale des préparations et **en ce que** le rapport de la substance A) à la substance B) est de 1 à 20, de manière particulièrement préférée de 1,5 à 15.

2. Utilisation de préparations cosmétiques ou dermatologiques présentant une teneur en
substance A) :
(1 R,2S,5R)-2-isopropyl-5-méthyl-N-(2-(pyridin-2-yl)éthylcyclohexanecarboxamide et/ou
(1 R,2S,5R)-N-(4-(cyanométhyl)-phényl)-2-isopropyl-5-méthylcyclohexanecarboxamide et
substance B) : menthoxypropanediol
**caractérisée en ce qu'**elles se trouvent sous forme d'émulsions H/E, **en ce qu'**elles contiennent 0,001-10% en poids, de manière particulièrement préférée 0,01-1% en poids, de substance A), par rapport à la composition totale des préparations et **en ce que** le rapport substance A) à substance B) est de 1 à 20, de manière particulièrement préférée de 1,5 à 15, pour produire une sensation de froid.
